# EUROPÄISCHE PATENTSCHRIFT

(11) **EP 3 463 273 B1**
(45) Veröffentlichungstag und Bekanntmachung des Hinweises auf die Patenterteilung: **11.11.2020**
(21) Anmeldenummer: 17726618.6
(22) Anmeldetag: 30.05.2017
(51) Int. Cl.: A61K 8/39, A61K 8/63, A61Q 90/00, A61K 31/08, A61K 31/19, A61P 3/04

(54) **NICHTIONISCHE TENSIDE ZUR REDUKTION VON FETTGEWEBE**
NONIONIC SURFACTANTS FOR REDUCTION OF ADIPOSE TISSUE
TENSIO-ACTIFS NONIONIQUES POUR LA RÉDUCTION D'UN TISSU GRAS

(30) Priorität: 30.05.2016 EP 16171904
(43) Veröffentlichungstag der Anmeldung: 10.04.2019
(73) Patentinhaber: CHEMISCHE FABRIK KREUSSLER & CO. GMBH, 65203 Wiesbaden (DE)
(72) Erfinder: TRAVERS, Stephan, 65187 Wiesbaden (DE); GLIEM, Petra, 65232 Taunusstein (DE); LOTTER, Matthias, 65201 Wiesbaden (DE); OTTO, Joachim, 65366 Geisenheim-Stephanshausen (DE)
(74) Vertreter: dompatent von Kreisler Selting Werner - Partnerschaft von Patent- und Rechtsanwälten mbB
(86) Internationale Anmeldenummer: PCT/EP2017/062956
(87) Internationale Veröffentlichungsnummer: WO 2017/207520

(56) Entgegenhaltungen:
- CA-A1- 2 872 279
- US-A- 5 164 320
- D. BOLTEN ET AL: "Solubility of Ibuprofen, Phytosterol, Salicylic Acid, and Naproxen in Aqueous Solutions", CHEMICAL ENGINEERING AND TECHNOLOGY, Bd. 36, Nr. 3, 6. März 2013 (2013-03-06), Seiten 426-434, XP055292206, DE ISSN: 0930-7516, DOI: 10.1002/ceat.201200510
- MCDIARMID JAMES ET AL: "Results from a Pooled Analysis of Two European, Randomized, Placebo-Controlled, Phase 3 Studies of ATX-101 for the Pharmacologic Reduction of Excess Submental Fat", AESTHETIC PLASTIC SURGERY, SPRINGER VERLAG, NEW YORK, NY, US, Bd. 38, Nr. 5, 2. Juli 2014 (2014-07-02), Seiten 849-860, XP035397473, ISSN: 0364-216X, DOI: 10.1007/S00266-014-0364-9 [gefunden am 2014-07-02]

## Beschreibung

Die vorliegende Erfindung betrifft eine Zusammensetzung zu Reduktion von Fettgewebe, die Verwendung dieser Zusammensetzung sowie ein Verfahren zur Reduktion von unerwünschtem Fettgewebe.

Zunehmende Fettleibigkeit in der Bevölkerung stellt ein deutliches Problem insbesondere der Industrieländer dar. Dies hat Auswirkungen auf die Kosten der Gesundheitssysteme. Gleichzeitig wird ein Schönheitsideal propargiert, welches einen sehr schlanken Körper als Optimum darstellt. Dabei werden insbesondere lokale Fettpölsterchen, wie beispielsweise im Bereich der Wangenknochen oder des Kinns bereits als unattraktiv angesehen.

Um nun überschüssiges Fett zu entfernen sind unterschiedliche Möglichkeiten bekannt. Eine verbesserte Form der Fettabsaugung ist beispielsweise in EP 1 733 692 A1 offenbart.

Um die Risiken einer Operation zu umgehen werden zunehmend Injektionen eingesetzt, die entsprechende Fettpölsterchen entfernen sollen. So offenbart beispielsweise WO 02/060410 A2 die Verwendung des Signalstoffes TNF-α (Tumornekrose-Faktor-a) in einer entsprechenden Injektion. Die Kombination aus einem Chelatbildner für Eisen, einem osmotisch wirksamen Mittel, Aminosäuren und Vitaminen ist in WO 2013/093947 A1 offenbart.

Aufgabe der vorliegenden Erfindung ist es eine Zusammensetzung zur Reduktion von Fettgewebe bereit zu stellen, die langanhaltend und wirksam ist, den Stand der Technik bereichert und eine Alternative zu den bekannten Zusammensetzungen bietet. Zudem soll es zu möglichst geringen Nebenwirkungen, wie beispielsweise allergischen Reaktionen oder ähnlichem kommen.

Gelöst werden die Aufgaben durch Zusammensetzungen gemäß dem Hauptanspruch. Vorteilhafte Ausführungsformen sind in den Unteransprüchen genannt. Weiterhin umfasst die Erfindung die Verwendung der Zusammensetzung sowie ein Verfahren zur Reduktion von Fettgewebe, wie in den nebengeordneten Ansprüchen dargestellt.

Es hat sich gezeigt, dass Tenside und insbesondere nichtionische Tenside geeignet sind, Fettgewebe von Menschen zu reduzieren und vorzugsweise zu entfernen. Eine solche Reduktion erfolgt durch Verabreichung einer wässrigen Lösung der Tenside unmittelbar in das Fettgewebe, so dass eine Zusammensetzung gemäß der vorliegenden Erfindung wenigstens ein Tensid und insbesondere wenigstens ein nichtionisches Tensid als aktive Substanz sowie Wasser als Lösungsmittel aufweist. Das Wasser ist dabei für entsprechende Anwendungen geeignet und ist insbesondere Wasser für Injektionszwecke.

Das Tensid ist Polidocanol. Polidocanol ist eine andere Bezeichnung für Lauromacrogol 400, welches der folgenden Formel entspricht:

C₁₂H₂₅-(O-CHᵣCH₂)ₙ-OH.

Üblicherweise nimmt n einen Durchschnittswert von 9 an. Synonyme Bezeichnungen für Polidocanol sind beispielsweise Laureth-9, Lauromacrogol 400, Macrogol-Laurylether (Ph. Eur.), Polyethylenglycol-Laurylether, Dodecylalkohol-poly-9-glykolether, Hydroxypolyethoxydedecan oder Polidocanol 600. Die CAS No. lautet 9002-92-0 oder 3055-99-0.

Der folgende Wirkmechanismus wird für die erfindungsgemäße Zusammensetzung angenommen: Die aktive Substanz bewirkt, dass Flüssigkeit aus der extrazellulären Matrix (ECM) oder Lymphflüssigkeit in die Fettzelle eindringen kann. Dies führt zu einer Zellschwellung, wodurch ein osmotischer Austausch stattfindet. Die Fettzelle gibt so ihren Inhalt, insbesondere Fettsäuren, wie beispielsweise Triglyzeride, und Zytoplasma zumindest teilweise bevorzugt vollständig frei. Die bisher in den Fettzellen gespeicherten Fettsäuren können dann aus dem Körper ausgeschieden werden. Dies führt zur gewünschten Reduktion des Fettgewebes.

Das Polidocanol weist erfindungsgemäß vorzugweise ein mittleres Molekulargewicht im Bereich von 500 bis 700, insbesondere von 570 bis 600 auf. Weiter vorzugsweise weist es einen Cloud-Point in Wasser von 80°C bis 90°C auf.

Sowohl Polidocanol als auch das Na-Salz der Dihydroxycholansäure werden bereits am und im Menschen für unterschiedliche Indikationen eingesetzt, so dass das Risiko hinsichtlich unerwünschter Wirkungen im menschlichen Körper so gering wie möglich ist. Negative Langzeitwirkungen sind aus der bisherigen Anwendungen im Wesentlichen nicht bekannt. Überraschenderweise hat sich nun eine neue Verwendung, nämlich die lokale Fettreduktion, gezeigt, so dass die vorliegende Erfindung ebenfalls die Verwendung von nichtionischen Tensiden und insbesondere von Polidocanol beziehungsweise des Na-Salzes von Dihydroxycholansäure zur lokalen Reduktion von Fettgewebe betrifft.

Die erfindungsgemäße Zusammensetzung kann ein Tensid, insbesondere ein nichtionisches Tensid, als aktive Substanz enthalten. Es ist auch möglich, dass sie 2, 3 oder mehrere unterschiedliche Tenside, insbesondere nichtionische Tenside enthält. Vorzugsweise enthält sie Polidocanol und/oder das Na-Salz der Dihydroxycholansäure. Die aktive Substanz ist Polidocanol. Der Anteil aktiver Substanz in der Zusammensetzung beträgt vorzugsweise 0,1 Gew.% bis 15 Gew.%, insbesondere 0,3 Gew.% bis 15 Gew.% oder bis 8 Gew.%, insbesondere 0,5 Gew.% bis 5 Gew.% oder bis 3 Gew.%, besonders bevorzugt 0,8 Gew.% bis 2 Gew.% oder bis 1 Gew.%. Eine zu hohe Tensidkonzentration kann die Zellmembran der Fettzellen zerstören, so dass der Tensidgehalt bevorzugt 8 Gew.% oder weniger, insbesondere 5 Gew.-% oder weniger beträgt. Der Anteil in Gew.% bezieht sich immer auf das Gesamtgewicht der kosmetischen Zusammensetzung, welches 100 Gew.% entspricht.

Neben der aktiven Substanz kann die wässrige Zusammensetzung weitere Hilfsstoffe aufweisen. Hilfsstoffe sind dabei solche Verbindungen, die die Wirkung gegebenfalls unterstützen oder die Stabilität der Zusammensetzung verbessern. Eine eigene Wirkung hinsichtlich der Fettentfernung kann diesen jedoch nicht zugeschrieben werden. Sie können jedoch eine eigene Wirkung aufweisen, wie beispielsweise eine Verringerung der Irritation des Gewebes, welches das zu entfernende Fettgewebe umgibt. Entsprechende Hilfsstoffe sind erfindungsgemäß insbesondere ausgewählt aus anionischen, kationischen oder zwitterionischen Tensiden, Vitaminen, Aminosäuren, Verzögerungsmitteln, Lösungsvermittlern sowie Mischung dieser.

Vitamine und Aminosäuren werden zugegeben, um insbesondere das dem Fettgewebe umliegende Gewebe mit Nährstoffen zu versorgen, so dass eine möglichst geringe Anzahl von Nebenwirkungen erreicht wird. Zudem stellt sich so nach der Behandlung ein attraktives Hautbild dar.

Lösungsvermittler im Sinne der vorliegenden Erfindung sind solche Stoffe, die das nichtionische Tensid im Lösungsmittel, also Wasser, stabil lösen, so dass es über einen längeren Zeitraum hinweg, von wenigstens einer Woche oder länger, insbesondere von zwei Wochen oder länger, insbesondere von vier Wochen oder länger, vorzugsweise von drei Monaten oder länger, insbesondere von sechs Monaten oder länger, bei Raumtemperatur stabil gelagert werden kann. Ein Lösungsvermittler sorgt zudem für eine homogene Verteilung der aktiven Substanz im Lösungsmittel. Gleichzeitig ist der Lösungsvermittler ein solcher Stoff, der mit dem Fettgewebe nicht wesentlich in Wechselwirkung tritt, also die Wirkung des aktiven Stoffs nicht beeinflusst. Gleichzeitig treten keine weiteren Nebenwirkungen auf.

Bevorzugt ist der Lösungsvermittler vorliegend ausgewählt aus Alkoholen, insbesondere aus einwertigen und/oder mehrwertigen Alkoholen. Mehrwertige Alkohole im Sinne der vorliegenden Erfindungen sind vorzugsweise zwei- und/ oder dreiwertige Alkohole. Besonders bevorzugt als Lösungsvermittler sind entsprechende zwei- und/oder dreiwertige Alkohole mit weniger als 10 Kohlenstoffatomen, insbesondere mit 2 bis 5 Kohlenstoffatomen (C-Atomen). Neben Kohlenstoff und Wasserstoff weisen die Alkohole zusätzlich zu den Hydroxygruppen keine weiteren Atome auf. Besonders bevorzugt ist der Lösungsvermittler ein Diol, also ein zweiwertiger Alkohol mit 2 bis 5 Kohlenstoffatomen. Diese sorgen für eine homogene, gleichmäßige Verteilung sowie eine Stabilisierung der aktiven Substanz im Lösungsmittel und gleichzeitig führt es zu keinerlei unerwünschten Nebenwirkungen. Dabei stellt der Lösungsvermittler selbst kein Lösungsmittel dar. Der Anteil an Lösungsmittel ist immer größer als der Anteil an Lösungsvermittler.

Ein Verzögerungsmittel im Sinne der vorliegenden Erfindung ist eine solche Substanz, die nach Verabreichung der Zusammensetzung in das Fettgewebe dafür sorgt, dass die aktive Substanz gleichmäßig über einen längeren Zeitraum hinweg freigesetzt wird. Hierdurch wird ein gleichmäßiger Wirkspiegel über eine gewünschte Dauer hinweg ermöglicht, wodurch eine besonders gute Fettentfernung ermöglicht wird. Das Verzögerungsmittel kann beispielsweise eine Mikrokapsel sein. Diese kann beispielsweise in Form einer Kern-Hülle-Kapsel vorliegen. Im Kern enthält eine solche Kapsel die Aktivsubstanz. Die Hülle besteht dabei aus einem Material, welches im Fettgewebe löslich ist, sich jedoch in der wässrigen Lösung selbst nicht auflöst. Hierdurch kann es zu einer verzögernden Freisetzung der aktiven Substanz erst am Ort, an dem sie wirken soll, kommen. Gleichzeitig ist ein Transport zum gewünschten Ort sichergestellt, ohne dass es zu einer großflächigeren Verteilung der Aktivsubstanz im Gewebe und damit zu einer verringerten Konzentration am gewünschten Wirkort führen kann. Mikrokapseln können auch Speckles sein. Hierbei handelt es sich um solche Partikel, in welchem eine Matrix vorliegt. In diese Matrix ist dann die Aktivsubstanz eingebunden. Sowohl die Matrix von Speckles als auch die Hülle von Kern-Hülle-Kapseln können aus an sich bekannten Materialien bestehen, wie beispielsweise und bevorzugt Polylactiden, Polyglycoliden, Polymilchsäuren, Polyglykolsäuren, Polyanhydriden, Polyorthoesthern, Polyetheresthern, Polycaprolactonen, Polyestheramiden, Polycarbonaten, Polycyanoacrylaten, Polyurethanen, Polyacrylaten und Mischungen oder Copolymeren dieser. Verzögerungsmittel können auch Alginate sein. Bevorzugt ist das Verzögerungsmittel eine Mikrokapsel, welche Alginate und/oder Poly(lactid-co-glycolid) als Matrix beziehungsweise Hülle-Material aufweist.

Um nun eine konstante Freisetzung der aktiven Substanz über einen langen Zeitraum zu ermöglichen, kann die erfindungsgemäße Zusammensetzung die aktive Substanz beispielsweise unmittelbar gelöst aufweisen. Weiterhin kann sie Mikrokapseln, die das Verzögerungsmittel sowie die Aktivsubstanz aufweisen, enthalten. Wird die Zusammensetzung in das Fettgewebe injiziert, ist zum einen eine unmittelbare Wirkung am Injektionsort sichergestellt. Durch das Verzögerungsmittel wird dann eine verzögerte Freisetzung über einen Zeitraum von wenigstens drei Tagen, vorzugsweise von fünf Tagen oder länger sichergestellt. Der Zeitraum, über welchen das nichtionische Tensid freigesetzt wird, kann durch die Wahl des Verzögerungsmittels individuell eingestellt werden.

Die vorliegende Erfindung betrifft weiterhin die Verwendung einer kosmetischen Zusammensetzung wie zuvor beschrieben zur Reduktion von Fettgewebe mittels Injektionslipolyse. Injektionslipolyse ist dabei die Verabreichung der Zusammensetzung mittels Injektion für die Behandlung lokaler Fettdepots. Lokale Fettdepots können sich dabei am ganzen Körper befinden, insbesondere werden hierdurch Doppelkinn, Hängebäckchen, Oberarme, Achseln, Bauch, Hüften, Reiterhosen, Knie, Fesseln, Fettansammlungen an Po, Brust und an der Innenseite der Oberschenkel reduziert. Auch bei Pseudo-Gynäkomastie oder beim sogenannten Stiernacken ist eine Verwendung der erfindungsgemäßen Zusammensetzung möglich.

In einer weiteren Ausführungsform betrifft die vorliegende Erfindung ein Verfahren zur Reduktion von Fettgewebe. Dieses umfasst die Verabreichung einer Zusammensetzung wie zuvor beschrieben in das zu behandelnde Areal. Das zu behandelnde Areal ist dabei das Gebiet eines Körpers, insbesondere eines menschlichen Körpers, in welchem sich eine lokale Fettansammlung befindet. Dies kann dabei alle Regionen des menschlichen Körpers betreffen. Insbesondere umfasst sind dabei Kinn, Wangen, Oberarme, Achseln, Bauch, Hüften, Knie, Fesseln, Po, Brust und/oder Oberschenkel. Dabei erfolgt die Verabreichung vorzugsweise mittels Injektion. Insbesondere erfolgt die Injektion in das Unterhautfettgewebe des zu behandelnden Areals. Somit kann die aktive Substanz unmittelbar ihre Wirkung entfalten. Durch das in einer bevorzugten Ausführungsform enthaltene Verzögerungsmittel können insbesondere in solchen Arealen, in denen größere Ansammlungen von Fettgeweben vorherrschen, größere Mengen der Zusammensetzung eingebracht werden und diese dort länger wirken.

Um eine Sicherstellung der Wirkung zu erhalten, ist eine Wiederholung der Verabreichung empfehlenswert. Diese sollte jedoch in einem gewissen zeitlichen Abstand erfolgen, damit der Körper ausreichend Zeit hat, die freigesetzten Fettsäuren aus dem Körper auszuscheiden. Weiterhin sollte sich das umliegende Gewebe des zu behandelnden Areals erholen können.

Auch wenn die aktiven Substanzen, Lösungsmittel und Hilfsstoffe einer ausführlichen Prüfung unterzogen werden, so kann es dennoch zu unerwünschten Reaktionen im Körper kommen. Damit diese möglichst gering gehalten werden, ist ein zeitlicher Abstand von wenigstens einer Woche einzuhalten. Insbesondere erfolgt die Verabreichung in einem zeitlichen Abstand von 7 bis 10 Tagen.

Weist die Zusammensetzung ein besonders wirksames Verzögerungsmittel auf, welches die Freisetzung über eine Zeitraum von 3 Tagen oder mehr, insbesondere von bis zu 7 Tagen ermöglicht, erfolgt die Verabreichung in einem zeitlichen Abstand von 18 bis 30 Tagen oder sogar einem längeren Abstand.

## Patentansprüche

1. Zusammensetzung zur Verwendung bei der Behandlung von Fettleibigkeit durch Reduktion von Fettgewebe mittels Injektionslipolyse umfassend
a) Polidocanol als nichtionisches Tensid in einem Anteil von 0,1 Gew.-% bis 15 Gew.-%, und
b) Wasser als Lösungsmittel.

2. Zusammensetzung nach Anspruch 1, **dadurch gekennzeichnet, dass** das Polidocanol ein mittleres Molekulargewicht im Bereich von 500 bis 700, insbesondere von 570 bis 600, und/oder einen Cloud-Point in Wasser von 80 °C bis 90 °C aufweist.

3. Zusammensetzung nach einem der Ansprüche 1 oder 2, **dadurch gekennzeichnet, dass** der Anteil an nichtionischem Tensid 0,3 Gew.-% bis 10 Gew.-% beträgt.

4. Zusammensetzung nach einem der Ansprüche 1 bis 3, weiterhin umfassend einen oder mehrere Hilfsstoffe ausgewählt aus Vitaminen, Aminosäuren Verzögerungsmitteln, Lösungsvermittlern und Mischungen dieser.

5. Zusammensetzung nach Anspruch 4, **dadurch gekennzeichnet, dass** der Lösungsvermittler ausgewählt ist aus einwertigen Alkoholen und/oder mehrwertigen Alkoholen, insbesondere aus zwei- und/oder dreiwertigen Alkoholen mit weniger als 10 C-Atomen, insbesondere mit 2 bis 5 C-Atomen.

6. Zusammensetzung nach Anspruch 4, **dadurch gekennzeichnet, dass** das Verzögerungsmittel eine Mikrokapsel ist, welche als Kern-Hülle-Kapsel oder als Speckles vorliegt.

7. Zusammensetzung nach Anspruch 6, **dadurch gekennzeichnet, dass** die Speckles eine Matrix aufweisen und diese Matrix und/oder die Hülle der Kern-Hülle-Kapseln Polylactiden, Polyglycoliden, Polymilchsäuren, Polyglykolsäuren, Polyanhydriden, Polyorthoesthern, Polyetheresthern, Polycaprolactonen, Polyestheramiden, Polycarbonaten, Polycyanoacrylaten, Polyurethanen, Polyacrylaten und Mischungen oder Copolymeren dieser aufweisen, insbesondere aus diesen bestehen.

8. Zusammensetzung nach Anspruch 6, **dadurch gekennzeichnet, dass** die Mikrokapsel Alginate und/oder Poly(lactid-co-glycolid) als Matrix der Speckles beziehungsweise Hülle-Material aufweist.

9. Zusammensetzung zur Verwendung bei der Behandlung von Fettleibigkeit nach einem der Ansprüche 1-8 durch Reduktion von Fettgewebe umfassend die Verabreichung der Zusammensetzung in das betreffende Areal mittels Injektion insbesondere in das Unterhautfettgewebe.

10. Zusammensetzung nach Anspruch 9, **dadurch gekennzeichnet, dass** die Verabreichung wiederholt erfolgt, insbesondere in einem zeitlichen Abstand von 7 bis 10 Tagen.

11. Zusammensetzung nach Anspruch 9, **dadurch gekennzeichnet, dass** die Verabreichung wiederholt erfolgt, insbesondere in einem zeitlichen Abstand von 18 bis 30 Tagen oder mehr.

## Claims

1. A composition for use in the treatment of obesity by reducing fatty tissue by injection lipolysis, comprising
a) polidocanol as a non-ionic surfactant in a content of from 0.1% to 15% by weight; and
b) water as the solvent.

2. The composition according to claim 1, **characterized in that** the polidocanol has an average molecular weight within a range of from 500 to 700, especially from 570 to 600, and/or a cloud point in water of 80 °C to 90 °C.

3. The composition according to either of claims 1 or 2, **characterized in that** the content of non-ionic surfactant is from 0.3% to 10% by weight.

4. The composition according to any of claims 1 to 3, further comprising one or more auxiliaries selected from vitamins, amino acids, retardants, solubilizers, and mixtures thereof.

5. The composition according to claim 4, **characterized in that** said solubilizer is selected from monohydric alcohols and/or polyhydric alcohols, especially from dihydric and/or trihydric alcohols with less than 10 carbon atoms, especially with 2 to 5 carbon atoms.

6. The composition according to claim 4, **characterized in that** said retardant is a microcapsule, which is in the form of a core-shell capsule or speckles.

7. The composition according to claim 6, **characterized in that** said speckles have a matrix, and that such matrix and/or the shell of said core-shell capsule comprise, especially consist of, polylactides, polyglycolides, polylactic acids, polyglycolic acids, polyanhydrides, polyorthoesters, polyether esters, polycaprolactones, polyester amides, polycarbonates, polycyanoacrylates, polyurethanes, polyacrylates, and mixtures or copolymers thereof.

8. The composition according to claim 6, **characterized in that** said microcapsule comprises alginates and/or poly(lactide-co-glycolide) as the speckle matrix or shell material.

9. The composition for use in the treatment of obesity according to any of claims 1 to 8 by reducing fatty tissue, comprising the administration of the composition into the area in question by injection, especially into the subcutaneous fat tissue.

10. The composition according to claim 9, **characterized in that** said administration is effected repeatedly, especially at intervals of from 7 to 10 days.

11. The composition according to claim 9, **characterized in that** said administration is effected repeatedly, especially at intervals of 18 to 30 days or more.

## Revendications

1. Composition à utiliser dans le traitement de l'obésité par la réduction de tissu adipeux au moyen de la lipolyse par injections, comprenant
a) du polidocanol comme tensioactif non ionique dans une proportion de 0,1 % en poids à 15 % en poids, et
b) de l'eau comme solvant.

2. Composition selon la revendication 1, **caractérisée en ce que** ledit polidocanol présente un poids moléculaire moyen compris entre 500 et 700, notamment entre 570 et 600, et/ou un point de trouble dans de l'eau de 80 °C à 90 °C.

3. Composition selon l'une des revendications 1 ou 2, **caractérisée en ce que** la proportion de tensioactif non ionique est de 0,3 % en poids à 10 % en poids.

4. Composition selon l'une quelconque des revendications 1 à 3, comprenant en outre un ou plusieurs agents auxiliaires choisis parmi des vitamines, acides aminés, retardateurs, solubilisants, et des mélanges de ceux-ci.

5. Composition selon la revendication 4, **caractérisée en ce que** ledit solubilisant est choisi parmi alcools monohydriques et/ou alcools polyhydriques, notamment parmi des alcools dihydriques et/ou trihydriques ayant moins de 10 atomes de carbone, notamment de 2 à 5 atomes de carbone.

6. Composition selon la revendication 4, **caractérisée en ce que** ledit retardateur est une microcapsule, qui se présente sous la forme d'une capsule de type noyau/coque ou de mouchetures.

7. Composition selon la revendication 6, **caractérisée en ce que** lesdites mouchetures présentent une matrice, et ladite matrice et/ou la coque des capsules de type noyau/coque comprennent, notamment consistent en, des polylactides, polyglycolides, poly(acides lactiques), poly(acides glycoliques), polyanhydrides, polyorthoesters, polyétheresters, polycaprolactones, polyesteramides, polycarbonates, polycyanoacrylates, polyuréthanes, polyacrylates, et des mélanges ou des copolymères de ceux-ci.

8. Composition selon la revendication 6, **caractérisée en ce que** ladite microcapsule contient des alginates et/ou du poly(lactide-co-glycolide) comme matrice des mouchetures ou matériau de la coque.

9. Composition à utiliser dans le traitement de l'obésité selon l'une quelconque des revendications 1 à 8 par la réduction de tissu adipeux, comprenant l'administration de ladite composition dans la région concernée par injection, notamment dans la graisse sous-cutanée.

10. Composition selon la revendication 9, **caractérisée en ce que** ladite administration est effectuée plusieurs fois, notamment dans un intervalle de temps de 7 à 10 jours.

11. Composition selon la revendication 9, **caractérisée en ce que** ladite administration est effectuée plusieurs fois, notamment dans un intervalle de temps de 18 à 30 jours ou plus.
